# EUROPEAN PATENT APPLICATION

(11) **EP 2 829 540 A1**
(43) Date of publication of application: **28.01.2015**
(21) Application number: 13178184.1
(22) Date of filing: 26.07.2013
(51) Int. Cl.: C07D 403/12

(54) **Synthesis of substituted aminopyridines**

(71) Applicant: Abbott Healthcare Products B.V., 1381 CP Weesp (NL); ABBOTT LABORATORIES, Abbott Park, IL 60064-3500 (US)
(72) Inventor: ACQUASALIENTE, Maurizio, ABBOTT PARK, 60064-6008, IL (US); LAGERWEIJ, Gert, 1381CP WEESP (NL); DESHPANDE, Mahendra, NORTHBROOK, 60062, IL (US); SHAH, Rajendra, NORTHBROOK, 60062, IL (US)
(74) Representative: Brunetti, Fabrizio

(57) **Abstract**

The present invention relates to a novel process for the manufacture of certain substituted aminopyridines having Formula (I) 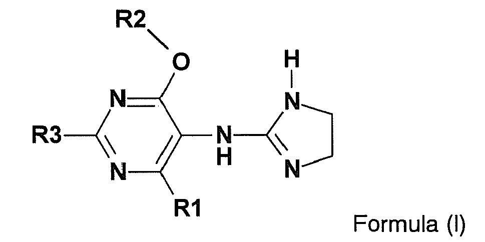 or a tautomer or salt thereof, wherein
R1 is selected from hydrogen, halogen, C₁₋₄alkoxy, C₁₋₄alklthio, C₁₋₄alkyl, C₃₋₅cycloalkyl, for example R1 is halogen such as chloro;
R2 is C₁₋₄alkyl, for example methyl, ethyl, i-propyl, n-propyl or butyl (any isomer), such as methyl; and
R3 is selected from hydrogen, C₁₋₄alkoxy, C₁₋₄alkylthio, C₁₋₄alkyl, C₃₋₅cycloalkyl, for example C₁₋₄alkyl such as methyl, ethyl, i-propyl, n-propyl or butyl (any isomer), for example methyl;
crystalline forms thereof as well as chemical intermediates suitable for use in performing the process.

## Description

The present disclosure relates to organic chemistry, in particular to a process for the preparation of certain substituted aminopyridines, such as moxonidine, their crystalline forms as well as certain chemical intermediates suitable for use in performing the process.

### Background

US4323570 discloses certain substituted aminopyridines, including moxonidine, and their preparation. 6-alkoxy-5-imidazolidinyl aminopyrimidines are prepared in US4323570 by reacting a corresponding 6-chloro-5-imidazolidinone intermediate with a suitable reagent such as a desired alcoholic sodium alkoxide. During the same step, any nitrogen protecting group possibly present on the 5-imidazolidinyl moiety (e.g. an alkanoyl protecting group) is concurrently removed. Later documents, such as EP0857483, discussing the preparation or use of similar 6-alkoxy-5-imidazolidinyl aminopyrimidines do not depart from the same synthetic strategy of US4323570 and still introduce the 6-alkoxy substituent close to the end of the process e.g. by means of a metallic alkoxide. However, this synthetic route is not fully satisfactory, especially for scaling-up to amounts suitable for clinical development or drug commercialization. Reasons may be found in poor selectivity and low yields. It is thus an object of the present disclosure to disclose a new and efficient synthetic route to prepare certain 6-alkoxy substituted aminopyrimidines, in particular moxonidine, with improved selectivity and yields, by using readily available or accessible building blocks under mild process conditions which are fully amenable to all purposes, including process scale-up. It is another object of the present disclosure to provide novel process intermediates suitable for use in the above process.

### Brief description of Figures

Figure 1 depicts the mass (MS) spectrum obtained for crude moxonidine free-base as obtained in example 4, batch number OSL-ABT-01-39;
Figure 2 depicts the mass (MS) spectrum obtained for crude moxonidine free-base as obtained in example 4, batch number OSL-ABT-01-43;
Figure 3 depicts an expanded section of the mass (MS) spectrum of Figure 2;
Figure 4 depicts the ¹³C NMR spectrum obtained for crude moxonidine free-base as obtained in example 4, batch number OSL-ABT-01-43;
Figure 5 depicts the ¹H NMR spectrum obtained for crude moxonidine free-base as obtained in example 4, batch number OSL-ABT-01-39;
Figure 6 depicts the ¹H NMR spectrum obtained for crude moxonidine free-base as obtained in example 4, batch number OSL-ABT-01-43.

### Disclosure

Unless otherwise indicated, "salt" is preferably a pharmaceutically acceptable salt.

Unless otherwise indicated, "room temperature" means about 20°C.

In one aspect, the present disclosure discloses a process for the manufacture of a substituted aminopyridine of Formula (I) 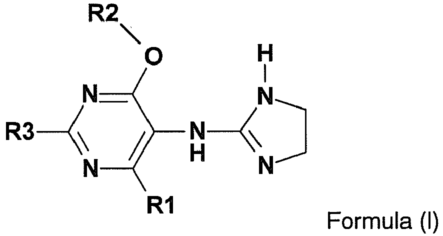 or a tautomer or salt thereof, wherein
R1 is selected from hydrogen, halogen, C₁₋₄alkoxy, C₁₋₄alklthio, C₁₋₄alkyl, C₃₋₅cyloalkyl, for example R1 is halogen, such as chloro;
R2 is C₁₋₄alkyl, for example methyl, ethyl, i-propyl, n-propyl or butyl (any isomer), such as methyl; and
R3 is selected from hydrogen, C₁₋₄alkoxy, C₁₋₄alkylthio, C₁₋₄alkyl, C₃₋₅cycloalkyl, for example C₁₋₄alkyl such as methyl, ethyl, i-propyl, n-propyl or butyl (any isomer), for example methyl;
said process comprising a step (i-a) of reacting a compound of Formula (Ia) 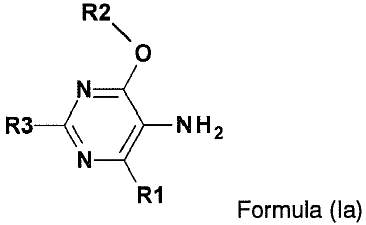 wherein R1, R2 and R3 are as defined above, with a compound of formula (Ib) 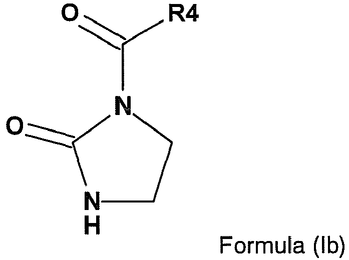 wherein R4 is C₁₋₃alkyl, such as methyl, ethyl or propyl, for example methyl, and
wherein step (i-a) is performed in a suitable solvent and in the presence of an effective amount of an acid reagent.

Step (i-a) corresponds to the reaction of Scheme 1, below:

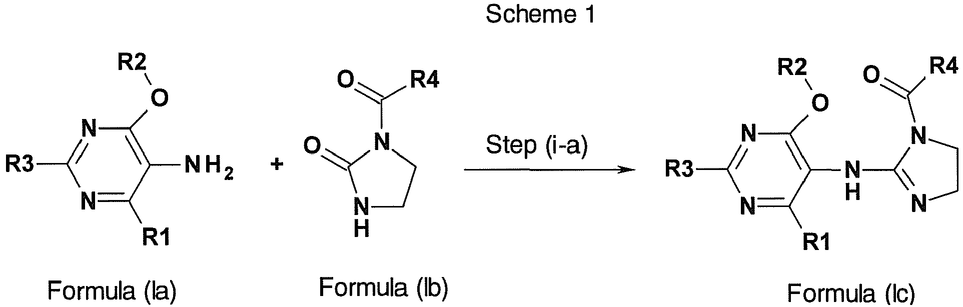

Compound of formula (Ic) may be obtained in any tautomer thereof.

Starting materials having Formula (Ia) and (Ib) are commercially available at an affordable cost (e.g. compound having CAS no. 88474-31-1 which corresponds to compound of Formula (IIa), or compound having CAS no. 5391-39-9 which corresponds to compound of Formula (IIb), both mentioned below).

In step (i-a), the compounds of Formula (Ia) and (Ib) may be used in about stoichiometric amounts.

Conveniently, the acid reagent of step (i-a) may be an acid, such as a strong acid. For example, the acid reagent may be a mineral and/or organic acid. Examples of acids may be gaseous HCl; a carboxylic acid, such as a carboxylic acid having formula RCOOH wherein R is hydrogen or optionally halogen substituted C₁₋₃ alkyl; H₂SO₄; and mixtures thereof. Conveniently, the acid reagent may be a chlorinating agent. For example, the chlorinating agent may be selected from SOCl₂, PCl₅, PCl₃, POCl₃, and mixtures thereof. Suitably, the chlorinating agent includes, such as consist of, POCl₃.

An effective amount of an acid reagent in step (i-a) is any amount that favours the coupling between compounds of Formula (Ia) and (Ib) as per Scheme 1. In step (i-a) the acid reagent may be present in an amount comprised between about 0.1 and 2 equivalents. Catalytic amounts of the acid reagent may be used for example when the acid reagent is not consumed during the reaction. Mineral and/or organic acids such as those disclosed above are suitable to be used as acid catalysts in step (i-a). Amounts comprised between about 0.1 and 1 equivalents, for example between about 0.1 and 0.2, or between about 0.1 and 0.3, or between about 0.1 and 0.4, and less than about 1, for example less than about 0.9 or less than about 0.8 or less than about 0.7, such as less than about 0.6, for example less than about 0.5 or less than about 0.4 equivalents are examples of catalytic amounts. Suitable catalytic amounts are those which are effective to favour the coupling of the reactants without concurrently affecting their reactivity (for example by protonating the primary amino group of the compound of Formula (Ia)). Alternatively, greater amounts of the acid reagent, such as excess amounts may be used. This may be expedient for example when the acid reagent is also used as solvent for step (i-a) and/or when the acid reagent is consumed during the reaction. For example, chlorinating agents such as those disclosed above are acid reagents that may be used in excess amount. POCl₃, which is a particularly preferred acid reagent for step (i-a), may also be used as solvent. Amounts comprised between about 0.5 and 2 equivalents, for example between about 0.7 and 2 equivalents, such as between about 1 and 2 equivalents, for example more than about 1, or more than about 1,2, or more than about 1.3, or more than about 1.4, or more than about 1.5 equivalents, and less than about 2, such as less than about 1.9 or less than about 1.8 equivalents are examples of excess amounts.

A suitable solvent for step (i-a) may be any inert organic solvent or a chlorinating agent as defined above (for example POCl₃). For example a suitable solvent may be a polar aprotic solvent and/or a non-polar solvent. Examples of suitable solvents are dimethyl sulfoxide, dimethylformammide, monochlorobenzene, dichloromethane, toluene, tetrahydrofuran, and mixtures thereof. Conveniently, the solvent may include, for example consist of, dimethyl sulfoxide (DMSO).

Step (i-a) may be performed at a temperature comprised between 20°C and the solvent boiling point. For example, when the solvent is DMSO, the temperature may be comprised between 50°C and 65°C, such as between 55°C and 60°C.

Step (i-a) may be performed for a time comprised between 12 and 72 hours. For examples, it may be performed for a time comprised between 36 and 60 hours, such as 48 hours.

When the acid reagent is used in amounts higher than catalytic amounts, for example higher than stoichiometric amounts such as in excess amounts as defined above, it may be convenient that step (i-a) is performed in the presence of an effective amount of a base. Bases, being hydrogen ion acceptors, may avoid unwanted side-reactions like protonation of the basic amine moiety of the compound of Formula (Ia) thereby preserving overall reaction yields. The use of a base is particularly advantageous when using an excess amount of acid reagent, for example when using reagents (like chlorinating agents) that generate hydrogen ions as "by-product" while the desired reaction proceeds. For example, it may be convenient to use an effective amount of a base when the acid reagent in step (i-a) is selected from SOCl₂, PCl₅, PCl₃, POCl₃, and mixtures thereof, such as POCl₃, and the acid reagent is used in an excess amount as defined above. An effective amount of a base is an amount that allows capturing the unnecessary hydrogen ions while preserving the desired effect of the acid reagent. Amounts comprised between about 0 and 2 equivalents, such as more than about 0.1, or more than about 0.5, for example more than about 0.8, or more than about 1, or more than about 1.5 equivalents, and less than about 2 equivalents, are examples of suitable base amounts. The base may include an alkali metal carbonate, such as anhydrous sodium carbonate.

Removal of the alkanoyl protecting group R4-C(O)→ can be achieved through any suitable technique known in the art to cleave amide bonds. Accordingly, the process presently disclosed may further include a step (ii-a) of reacting the product obtained in step (i-a) with a reagent suitable to remove the R4-C(O)→ moiety.

For example, the process presently disclosed may further include a step (ii-a) of reacting the product obtained in step (i-a) with an acid alcohol solution in a suitable solvent, thereby obtaining an acid addition salt of a compound of Formula (I). If the acid alcohol solution is an HCl alcohol solution, step (ii-a) corresponds to the reaction of Scheme 2, below:

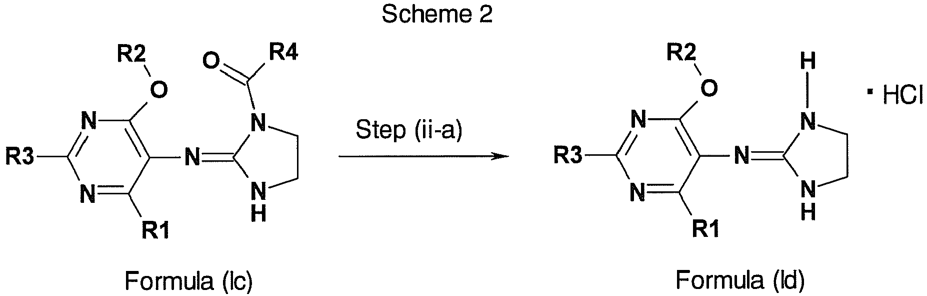

In step (ii-a) the acid alcohol solution may be an isopropanol-hydrochloride acid solution. The suitable solvent may be an aliphatic alcohol, such as methanol, n-propanol, butanol or mixtures thereof.

Conveniently, product of step (i-a) - i.e. the compound of Formula (Ic) - may be directly used in step (ii-a). This may mean that no purification step is performed on the product of step (i-a) before using it in step (ii-a).

The process presently disclosed may further include a step (iii-a) of purifying the product of step (ii-a). For example, if the product of step (ii-a) is obtained as an acid addition salt, step (iii-a) suitably includes the steps of obtaining the product of step (ii-a) in a free base form, and crystallizing said free base from a polar solvent. In case the product of step (ii-a) is a hydrochloride salt, step (iii-a) may correspond to the reaction of Scheme 3 below (wherein only one of the possible tautomers of Formula (I) is represented):

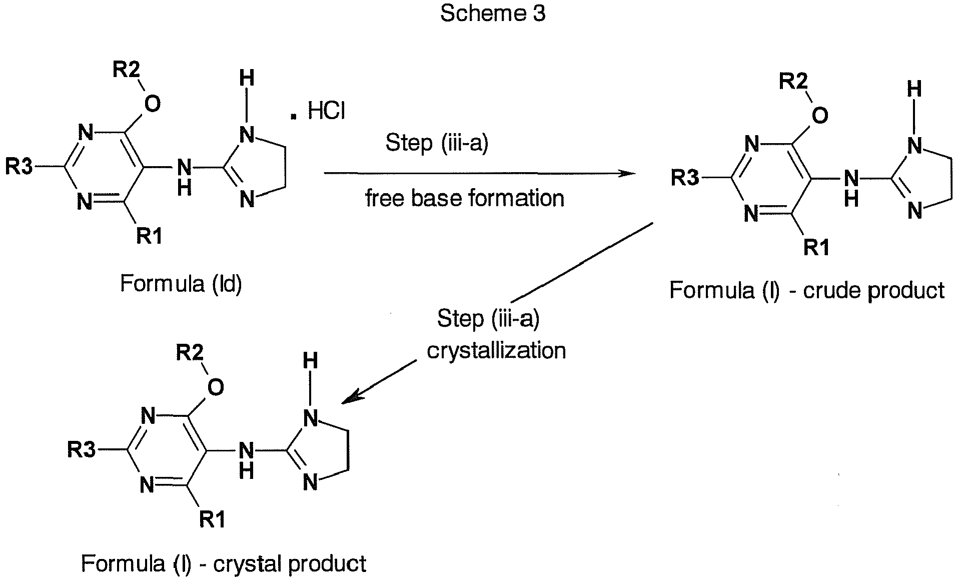

The achievement of the free base of the compound may include dissolving the product of step (ii-a) in water at a pH comprised between 8 and 10, for example pH 9 (if necessary, pH adjustment can be achieved through the addition of a base such as NaOH), and extracting the free base with a suitable organic solvent, such as any organic solvent which is not miscible with water. The solvent may for example include ethyl acetate and/or isopropyl acetate.

The crystallization step may include dissolving the free base obtained in the upstream step in a C₁₋₄ aliphatic alcohol solvent, such as methanol, ethanol, isopropanol or mixtures thereof, or triturating said free base in a ketone solvent. If dissolving the compound in a C₁₋₄ aliphatic alcohol solvent is chosen, the step conveniently includes dissolving the free base in a C₁₋₄ aliphatic alcohol solvent such as methanol, ethanol, isopropanol or mixtures thereof, and in the presence of activated carbon and/or carbon black. Though not being necessary, the use of carbon may allow removing unwanted impurities (which may be perceived as a discoloration of the crystallization medium). It is for example possible crystallizing the free base by dissolving the same in methanol at reflux and in the presence of activated carbon. Alternatively, the crystallization step may include triturating the free base obtained in the upstream step in a ketone solvent, such as acetone, at a temperature which may be comprised between about room temperature and about 50°C, such as at about room temperature or at about 40°C. The crystallization may be completed for example by letting the desired crystals to form at an effective temperature, such as room temperature, and finally collecting the formed crystals.

The process presently disclosed is particularly suitable for the manufacture of a compound of Formula (II) 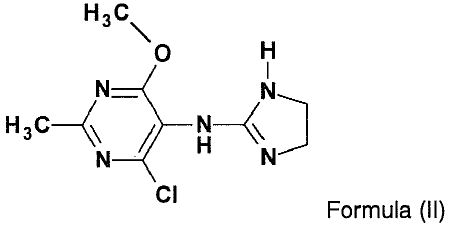 or a tautomer or salt thereof, in particular the preparation of the free base of a compound of Formula (II) or a tautomer thereof, which is 4-chloro-6-methoxy-2-methyl-5-(2-imidazolin-2-yl)aminopyrimidine, also known as moxonidine. In this case, the process comprises a step (i-b) of reacting a compound of Formula (IIa) 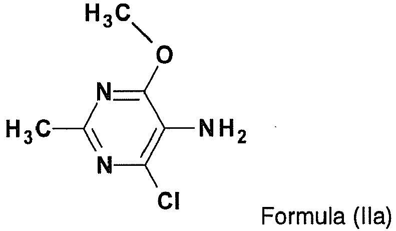 with a compound of formula (Ib) 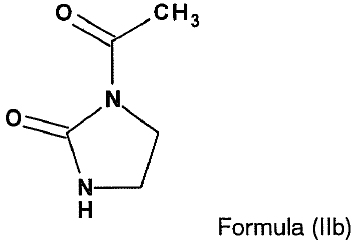 wherein step (i-b) is performed in a suitable solvent and in the presence of an effective amount of an acid reagent.

Step (i-b) is equivalent to step (i-a). Features and preferred options disclosed above in connection with step (i-a) may be applicable to its equivalent step (i-b). In particular this holds for the preferred use of DMSO as solvent, the use of POCl₃ as preferred acid reagent and the optional but preferred concurrent use of a base in step (i-b) as acid acceptor in order to further improve the reaction of compounds of Formula (IIa) and (IIb).

The process for the preparation of a compound of Formula (II) or a tautomer or salt thereof may further include a step (ii-b) of reacting the product obtained in step (i-a) with a reagent suitable to remove the CH₃-C(O)→ moiety. For example, the process may further include a step (ii-b) of reacting the product obtained in step (i-b) with an acid alcohol solution in a suitable solvent, thereby obtaining an acid addition salt of a compound of Formula (II). Step (ii-b) is equivalent to step (ii-a). Features and preferred options disclosed above in connection with step (ii-a) may be applicable to its equivalent step (ii-b). In particular this holds for the optional direct use of the product obtained in step (i-b) into step (ii-b), the use of an isopropyl alcohol/HCl solution as reaction medium to obtain an acid addition salt of the compound of Formula (II) and the concurrent removal of the alkanoyl protecting group present on the imidazolidinyl moiety.

The process for the preparation of a compound of Formula (II) or a tautomer or salt thereof may further include a step (iii-b) of purifying the product of step (ii-b). For example, if the product of step (ii-b) is obtained as an acid addition salt, step (iii-b) suitably includes the steps of obtaining the product of step (ii-a) in a free base form, and crystallizing said free base from a polar solvent. Step (iii-b) is equivalent to step (iii-a). Features and preferred options disclosed above in connection with step (iii-a) may be applicable to its equivalent step (iii-b). In particular this holds for the possibility to obtain a crude free base of the compound of Formula (II) by dissolving the product of step (ii-b) in water at a basic pH and subsequently crystallizing the desired product of Formula (II) by dissolving the free base in a C₁₋₄ aliphatic alcohol solvent, such as in methanol at reflux, optionally in the presence of carbon black or activated carbon, or triturating said free base in a ketone solvent.

Accordingly, the process for the manufacture of a compound of Formula (II) may be represented in the following Scheme 4:

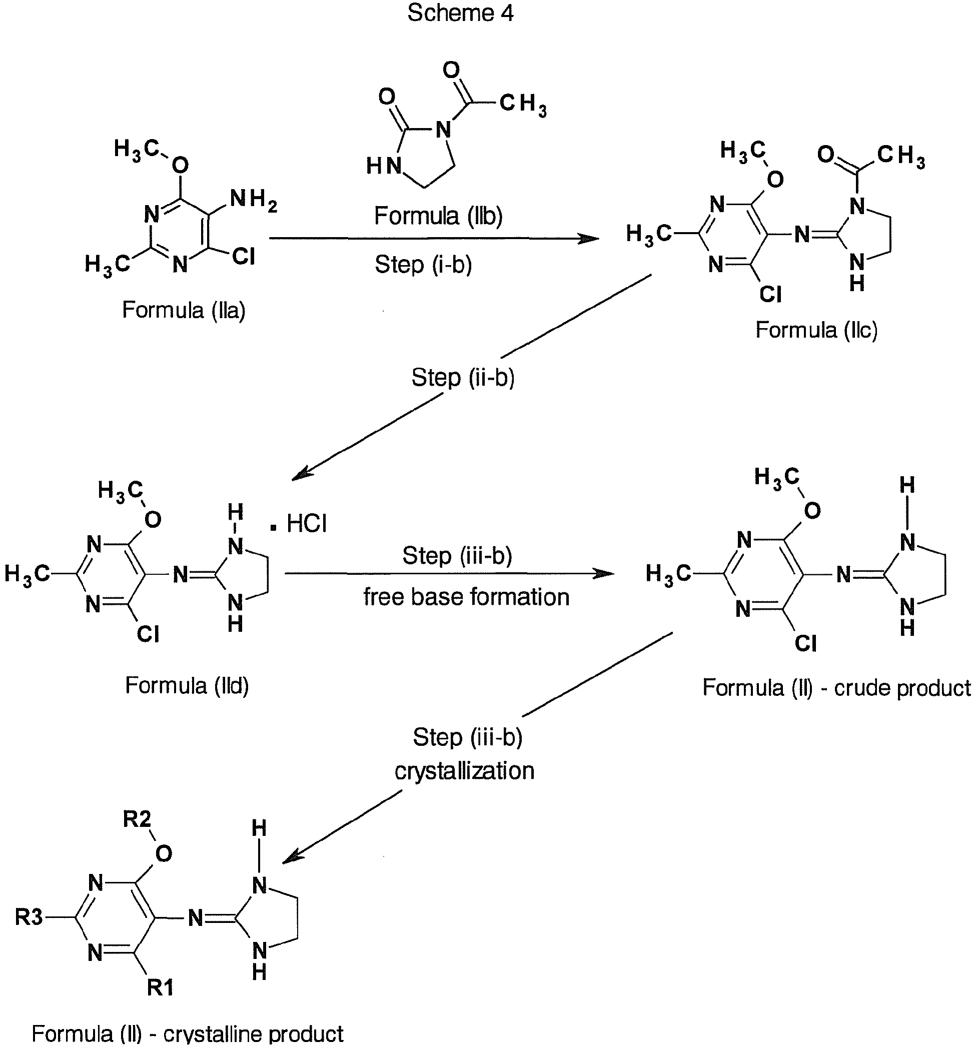

For example, the instant disclosure discloses a process for the manufacture of a compound of Formula (II) 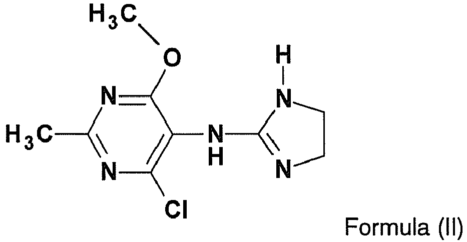 or a tautomer thereof, said process comprising:
- a step (i-c) of reacting a compound of Formula (IIa) 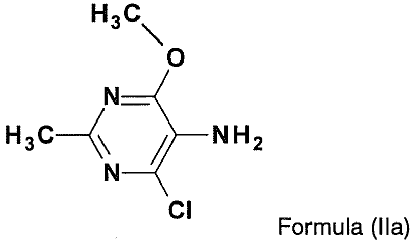 with a compound of formula (Ib) 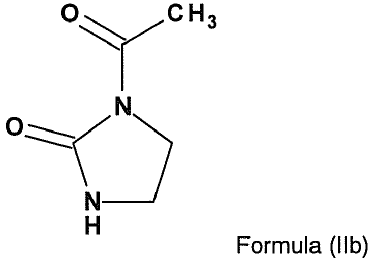 in dimethyl sulfoxide, in the presence of an effective amount of POCl₃ and an effective amount of anhydrous sodium carbonate;
- a step (ii-c) of directly reacting the product obtained in step (i-c) with a isopropanol-hydrochloride acid solution, thereby obtaining the chlorhydrate salt of the compound of Formula (II),
- a step (iii-c) of
   ○ dissolving the product of step (ii-c) in water at a pH of about 9 thereby obtaining the product of step (ii-c) in a free base form, and subsequently extracting said free base with ethyl acetate and/or isopropyl acetate,
   ○ subsequently dissolving said free base form in methanol at reflux and in the presence of activated carbon, and
   ○ collecting crystals of compound of Formula (II) or a tautomer thereof.

Accordingly, in one aspect the process for the manufacture of a compound of Formula (II) may be represented by steps (i-c) to (iii-c), as per the following Scheme 5:

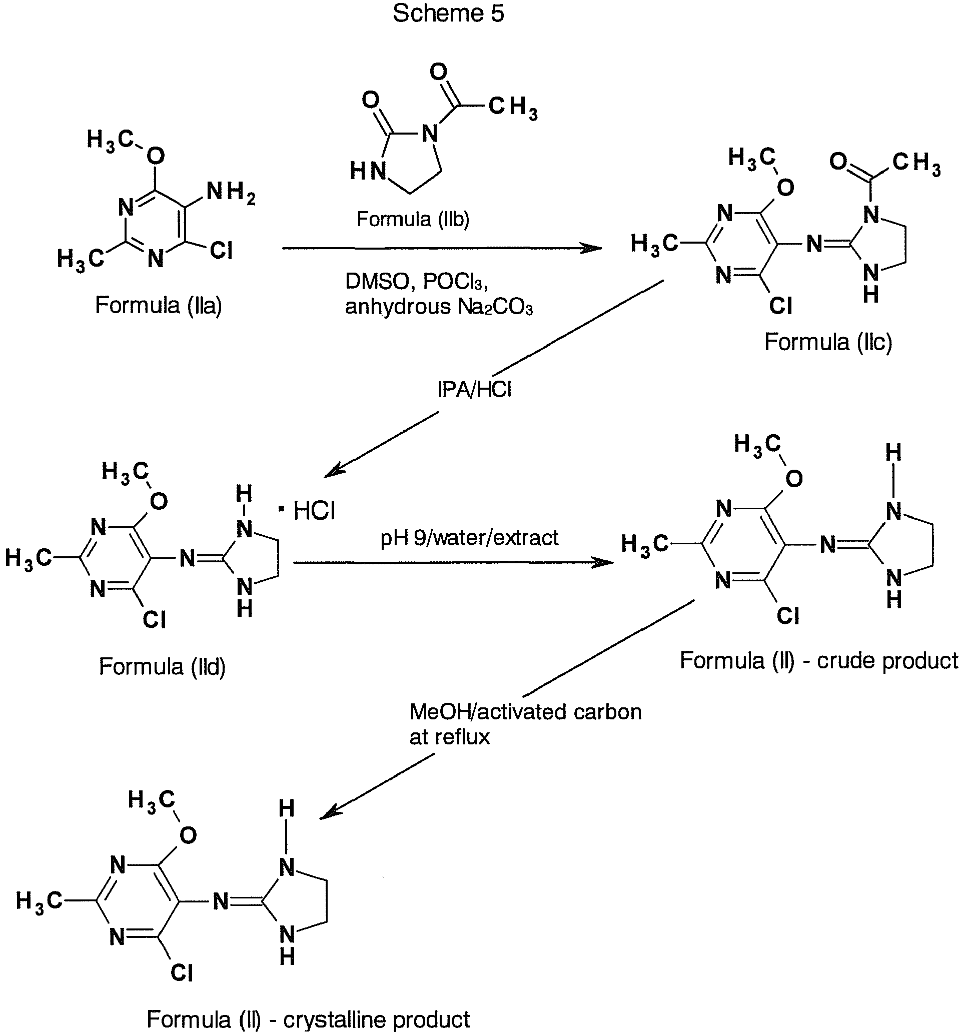

In one aspect, the present disclosure discloses a process for the manufacture of a crystalline form of a compound of Formula (II) or a tautomer thereof, 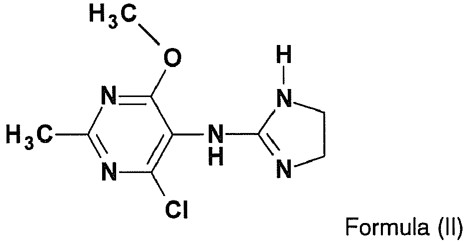 said process comprising the steps of
- dissolving a compound of Formula (II) in a C₁₋₄ aliphatic alcohol solvent or triturating the same in a ketone solvent, and
- collecting crystals of compound of Formula (II) or a tautomer thereof.

Features disclosed above in the context of crystallization steps (iii-a) or (iii-b) may be relied upon for the preparation of a crystalline form of a compound of Formula (II). In particular, examples of suitable C₁₋₄ aliphatic alcohol solvents are methanol, ethanol and isopropanol, preferably methanol. Similarly, it is preferred to use C₁₋₄ aliphatic alcohol solvents in the presence of an activated carbon and/or carbon black. Similarly, the preferred ketone solvent is acetone and the triturating step may be performed at a temperature conveniently comprised between room temperature and about 50°C. Finally, the crystallization may be obtained for example by letting the desired crystals to form at room temperature and finally collecting the formed crystals.

In one aspect, the present disclosure discloses a compound having formula (Ic) 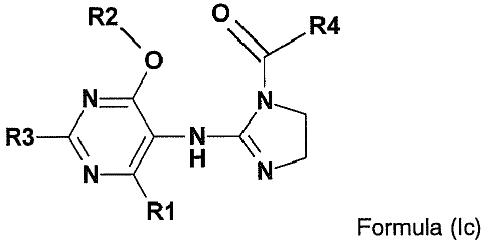 or a tautomer or salt thereof, wherein R1, R2, R3 and R4 are as defined above. For example, a preferred compound of formula (Ic) is a compound wherein R1 and R3 are as defined above and both R2 and R4 are methyl.

Compounds of Formula (Ic) are valuable intermediates in the processes presently disclosed.

A preferred compound of Formula (Ic) has Formula (IIc) 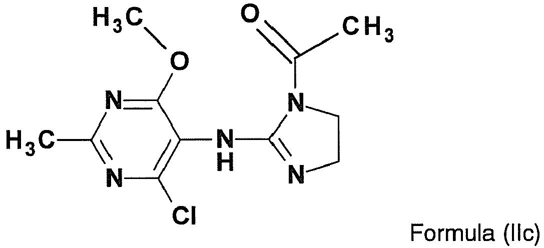 or a tautomer or salt thereof.

In one aspect, the present disclosure discloses the use of a compound of Formula (Ia) as intermediate in a process for the manufacture of a compound of Formula (I). For example, the present disclosure discloses the use of a compound of Formula (IIa) as intermediate in a process for the manufacture of a compound of Formula (II).

Further embodiments and advantages of the present invention will become apparent to a skilled reader in light of the examples provided below.

### Examples

### Example 1 (comparative): coupling of 5-amino-4-mehtoxy-6-chloro-3-methyl-pyrimidine and N-acetylimidazolidinone in the presence of an excess amount of acid catalyst but without a base.

5-amino-4-mehtoxy-6-chloro-3-methyl-pyrimidine (200mg, 1.25mmol) and N-acetylimidazolidinone (218.5mg, 1.22 mmol) were charged in 50mil flask. The flask was placed in ice-salt-acetone mixture and POCl₃ was added dropwise keeping a temperature below 5°C (due to the very exothermic addition). After the addition is over the ice bath was removed and the reaction mixture was stirred at 55°C under nitrogen for 48 hrs. The reaction mixture was checked by HPLC. The reaction didn't occur. Although it is not intended to be bound by any theory, it is believed that as POCl₃ reacted with N-acetylimidazolidinone, HCl gas was formed which protonated 5-amino-4-methoxy-6-chloro-3-methyylpyrimidine making 5-amino-4-methoxy-6-chloro-3-methyylpyrimidine unreactive.

### Example 2: coupling of 5-amino-4-mehtoxy-6-chloro-3-methyl-pyrimidine and N-acetylimidazolidinone in the presence of an excess amount of acid catalyst and Na₂CO₃ as a base

5-amino-4-methoxy-6-chloro-3-methyl-pyrimidine (2.0grams, 11.5mmol) and N-acetylimidazolidinone (2.0 grams, 11,5mmol) were dissolved in anhydrous DMSO and anhydrous sodium carbonate (3.75 grams, 35.4mmol, 2.0 equivalents) was added. The reaction flask was cooled in ice-salt-acetone mixture and POCl₃ was added dropwise in 1 hour keeping temperature below 5°C. After the addition the ice bath was removed and the flask was stirred at 58-60°C under nitrogen for 2 days. A small sample was taken and analysed by HPLC. There was no starting material and the product was formed. The anhydrous Na₂CO₃ neutralized the HCl gas in situ as it formed. Workup: The reaction mixture was quenched by slowly pouring in crushed ice. The product precipitated and the product was filtered and dried under vacuum. Without any further purification the product was used in the next step to generate crude moxonidine. Yield: 2.64 grams. Mass spec.: 283 (m+)

### Example 3: coupling of 5-amino-4-mehtoxy-6-chloro-3-methyl-pyrimidine and N-acetylimidazolidinone in the presence of an excess amount of acid catalyst and Na₂CO₃ as a base - scale up alternative

5-amino-4-methoxy-6-chloro-3-methyl-pyrimidine (20.0grams, 115mmol) and N-acetylimidazolidinone (20.0 grams, 115mmol) were dissolved in anhydrous DMSO and anhydrous sodium carbonate (37.5 grams, 35.4mmol, 2.0 equivalents) was added. The reaction flask was cooled in ice-salt-acetone mixture and POCl₃ was added dropwise in 2.5 hour keeping temperature below 5°C. After the addition the ice bath was removed and the flask was stirred at 58-60°C under nitrogen for 2 days. A small sample was taken and analysed by HPLC. There was no starting material and the product was formed. Workup: The reaction mixture was quenched by slowly pouring in crushed ice. The product precipitated and the product was filtered and dried under vacuum. Without any further purification the product was used in the next step to generate crude moxonidine. Yield: 31.7 grams (82.9%). Mass spec.: 283 (m+)

### Example 4: formation of 4-chloro-6-methoxy-2-methyl-5-(2-imidazolin-2-yl)aminopyrimidine hydrochloride (crude moxonidine hydrochloride) and subsequent preparation of 4-Chloro-6-methoxy-2-methyl-5-(2-imidazolin-2-yl)aminopyrimidine (crude moxonidine free base).

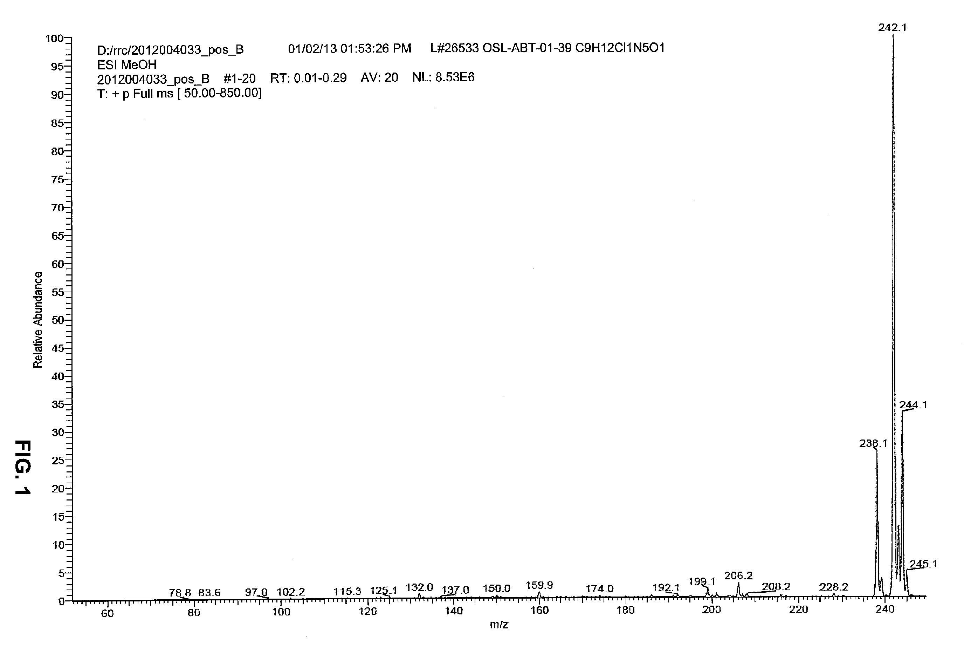

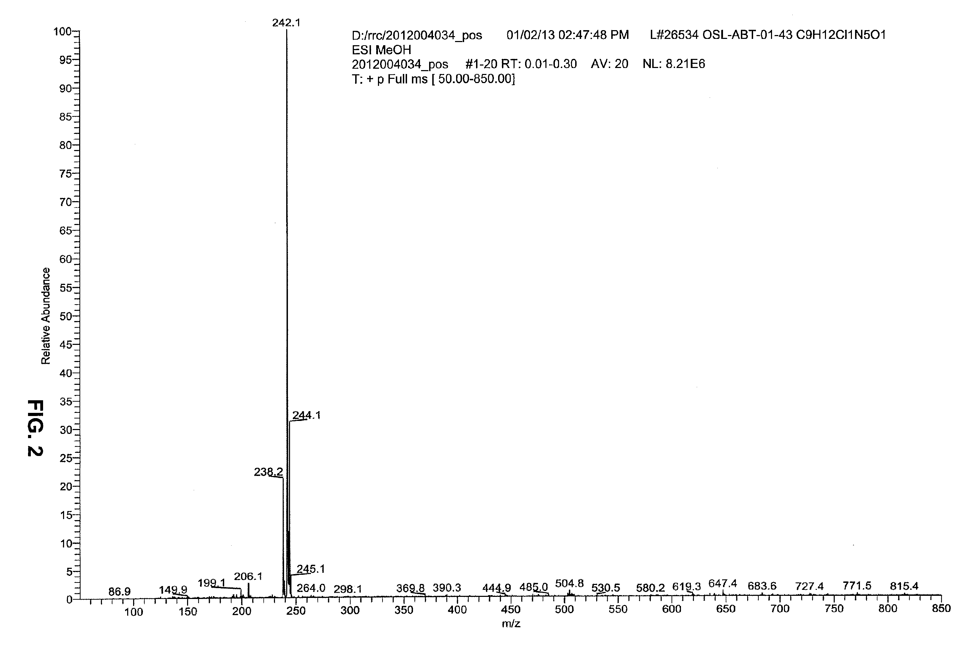

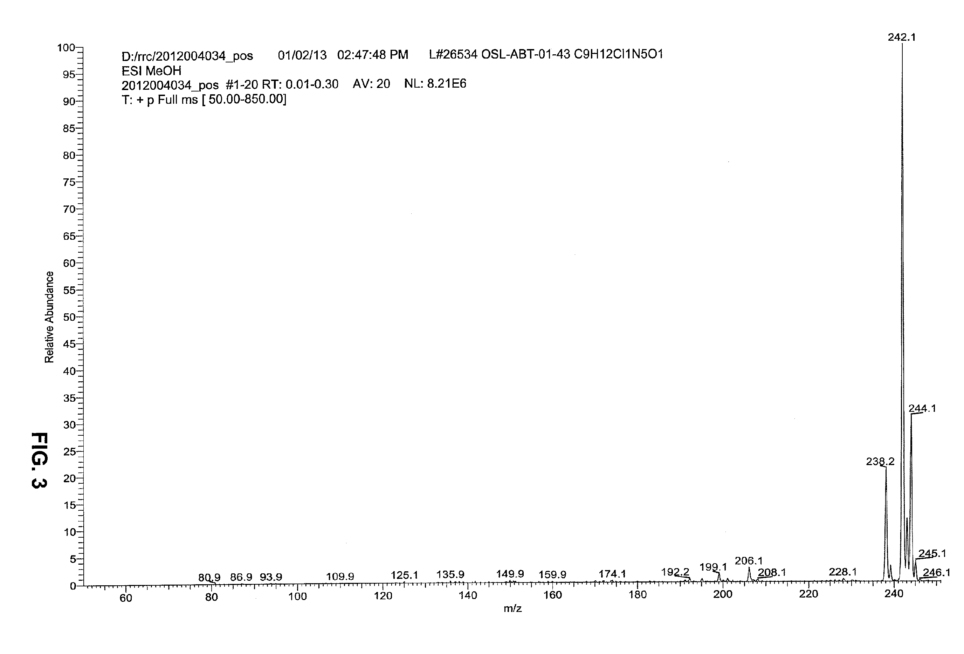

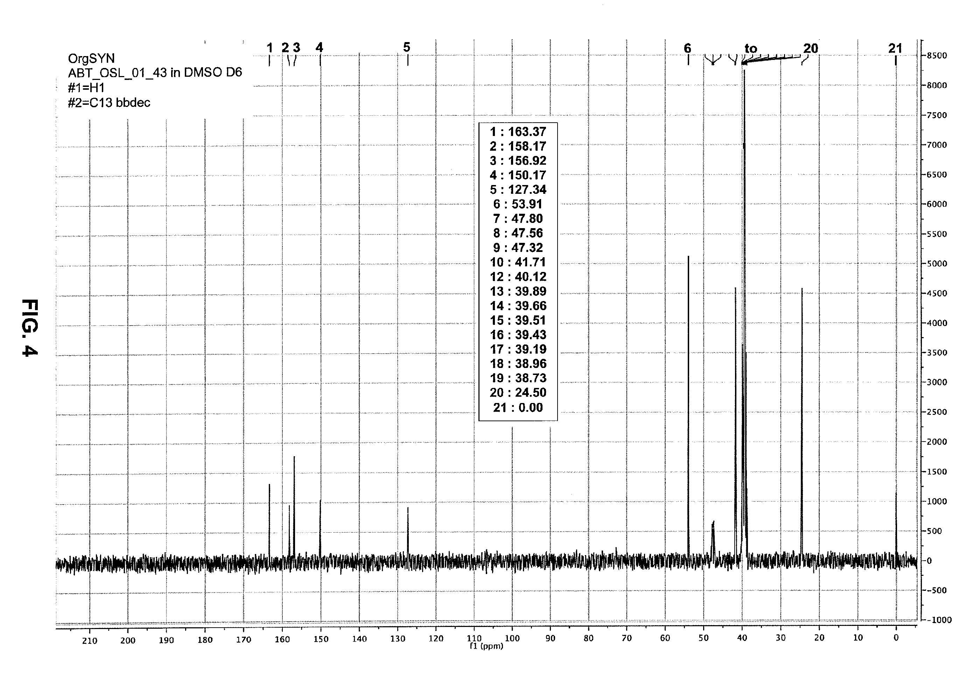

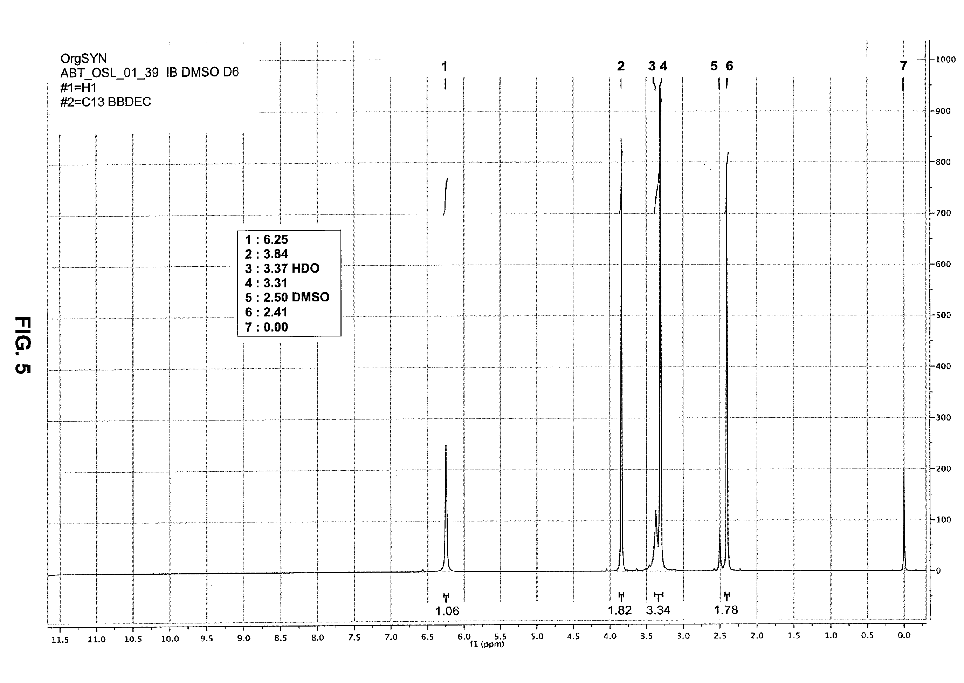

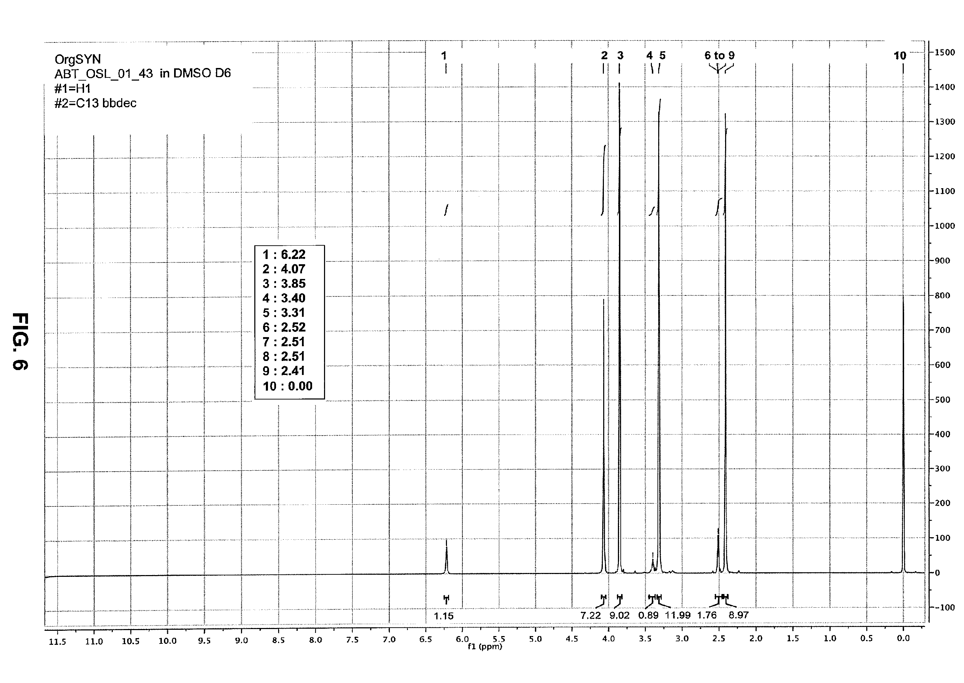

5 grams of the product obtained in example 3 was taken in 20ml isopropanol and isopropyl alcohol-HCl (10ml) was added. The resulting mixture was stirred at ambient temperature for 8 hours. The hydrochloride salt was precipitated and was then filtered and dried. The salt was dissolved in water and basified to pH 9.0. The product was extracted in ethyl acetate. Organic layer containing product was washed by water, brine solution and dried over anhydrous Na₂SO₄. Sodium sulfate was filtered off the solvent was distilled out to get the crude 4-Chloro-6-methoxy-2-methyl-5-(2-imidazolin-2-yl)aminopyrimidine (free base). Yield: 2.6 grams (72%). Mass spec.: 242 (m+) (see spectra of Fig. 1 to 3). NMR: see Fig. 4 for 13C NMR spectrum and Fig. 5-6 for 1 H NMR spectra.

### Example 5: formation of 4-chloro-6-methoxy-2-methyl-5-(2-imidazolin-2-yl)aminopyrimidine hydrochloride (crude moxonidine hydrochloride) and subsequent preparation of 4-Chloro-6-methoxy-2-methyl-5-(2-imidazolin-2-yl)aminopyrimidine (crude moxonidine free base).

The procedure disclosed in Example 4 was re-run to obtain a second, confirmatory batch of the product (batch OSL-ABT-01-41). 5 grams of the product obtained in example 3 was taken in 20ml isopropanol and IPA-HCl (10ml) was added. The resulting mixture was stirred at ambient temperature for 8 hours. The hydrochloride salt was precipitated and was then filtered and dried. The salt was dissolved in water and basified to pH 9.0. The product was extracted in ethyl acetate. Organic layer containing product was washed by water, brine solution and dried over anhydrous Na2SO4. Sodium sulfate was filtered off the solvent was distilled out to get crude 4-Chloro-6-methoxy-2-methyl-5-(2-imidazolin-2-yl)aminopyrimidine (free base). Yield: 2.6 Grams (72%). Mass spec.: 242 (m+)

### Example 6: purification of 4-chloro-6-methoxy-2-methyl-5-(2-imidazolin-2-yl)aminopyrimidine (crude moxonidine free base) via column chromatography.

An attempt was made to purify 4-Chloro-6-methoxy-2-methyl-5-(2-imidazolin-2-yl)aminopyrimidine by column chromatography using 30% ethyl acetate in hexane. The process, though successful, turned out to be very time consuming and poorly practical for application on a large scale.

### Example 7: purification of 4-chloro-6-methoxy-2-methyl-5-(2-imidazolin-2-yl)aminopyrimidine (crude moxonidine free base) via crystallization (runs 1 to 10).

Various samples of equal weight of crude 4-Chloro-6-methoxy-2-methyl-5-(2-imidazolin-2-yl)aminopyrimidine were crystallized under conditions (1) to (9) as defined below. Results in terms of recovery (expressed as % by weight over the original sample weight) and purity of finally crystallized 4-Chloro-6-methoxy-2-methyl-5-(2-imidazolin-2-yl)aminopyrimidine are reported in Table 1. Carbon filtration, though not being necessary, was adopted in certain cases to reduce colour of crystallization solution.

Crystallization conditions:
1 = methanol at reflux in the presence of activated carbon (e.g. Darco);
2 = ethanol at room temperature in the presence of activated carbon (e.g. Darco);
3 = ethanol at 60°C in the presence of carbon black (e.g. Lampblack);
4 = ethanol at 60°C in the presence of activated carbon (e.g. Darco);
5 = isopropanol at room temperature in the presence of activated carbon (e.g. Darco);
6 = isopropanol at room temperature in the presence of carbon black (e.g. Lampblack);
7 = isopropanol at room temperature in the presence of both carbon black (e.g. Lampblack) and activated carbon (e.g. Darco);
8 = crude 4-Chloro-6-methoxy-2-methyl-5-(2-imidazolin-2-yl)aminopyrimidine triturated in acetone at room temperature;
9 = crude 4-Chloro-6-methoxy-2-methyl-5-(2-imidazolin-2-yl)aminopyrimidine triturated in acetone at 40°C.

**Results - Table 1**

| | 1 | 2 | 3 | 4 | 5 | 6 | 7 | 8 | 9 |
|---|---|---|---|---|---|---|---|---|---|
| Purity | >99% | 98.2% | 98.01% | 98.01% | 98.33% | 98.2% | 98.12% | 98.22% | 98.04% |
| Recovery | 89% | 86% | 85% | 84.9% | 84.6% | 84.1% | 85.6% | 84.3% | 84.7% |

The above results support a preference for crystallizing in methanol at reflux and in the presence of activated carbon (Darco).

## Claims

1. A process for the manufacture of a compound of Formula (I) 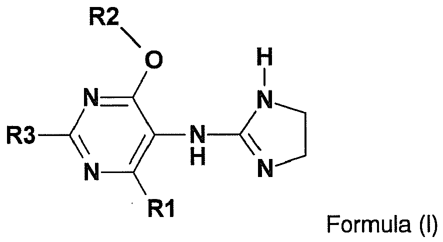 or a tautomer or salt thereof, wherein
R1 is selected from hydrogen, halogen, C₁₋₄alkoxy, C₁₋₄alklthio, C₁₋₄alkyl, C₃₋₅cycloalkyl;
R2 is C₁₋₄alkyl; and
R3 is selected from hydrogen, C₁₋₄alkoxy, C₁₋₄alkylthio, C₁₋₄alkyl, C₃₋₅cycloalkyl;
said process comprising a step (i-a) of reacting a compound of Formula (Ia) 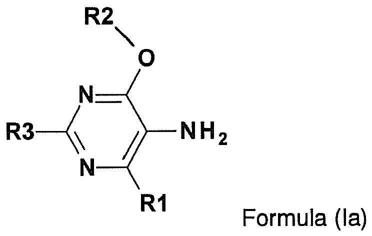 wherein R1, R2 and R3 are as defined above, with a compound of formula (Ib) 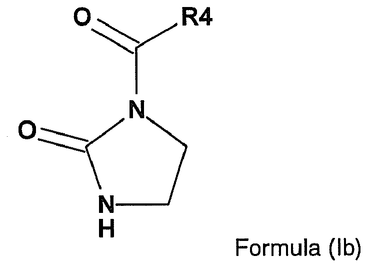 wherein R4 is C₁₋₃alkyl, and
wherein step (i-a) is performed in a suitable solvent and in the presence of an effective amount of an acid reagent.

2. The process according to claim 1, wherein
- the compound of Formula (I) has Formula (II) 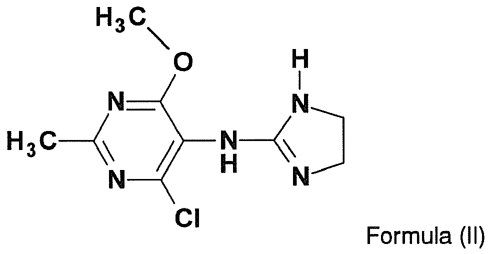 or a tautomer or salt thereof,
- the compound of Formula (Ia) has Formula (IIa) 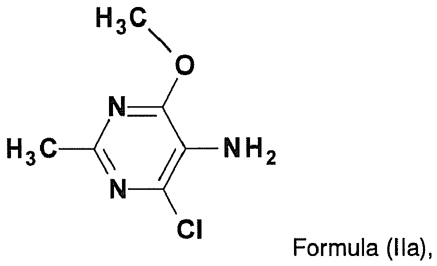 and
- the compound of Formula (Ib) has Formula (IIb)

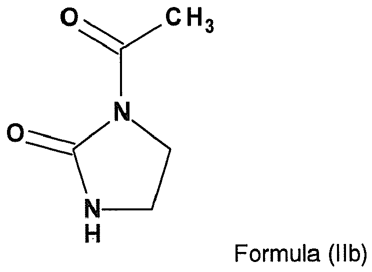

3. The process according to claims 1 or 2, wherein the acid reagent is a chlorinating agent.

4. The process according to claim 3, wherein the chlorinating agent is selected from the group consisting of SOCl₂, PCl₅, PCl₃, POCl₃, and mixtures thereof.

5. The process according to any one or more of claims 1 to 4 wherein step (i-a) is performed also in the presence of an effective amount of a base.

6. The process according to any one or more of claims 1 to 5, further including a step (ii-a) of reacting the product obtained in step (i-a) with a reagent suitable to remove the R4-C(O)→ moiety.

7. The process according to claim 6, wherein step (ii-a) includes reacting the product obtained in step (i-a) with an acid alcohol solution in a suitable solvent, thereby obtaining an acid addition salt of a compound of Formula (I).

8. The process according to claims 6 or 7, wherein the product of step (i-a) is directly used in step (ii-a).

9. The process according to any one or more of claims 1 to 8, further including a step (iii-a) of purifying the product of step (ii-a).

10. The process according to claim 9, wherein step (iii-a) includes the steps of obtaining the product of step (ii-a) in a free base form, and crystallizing said free base from a polar solvent.

11. The process according to claims 1 or 2 for the manufacture of a compound of Formula (II) 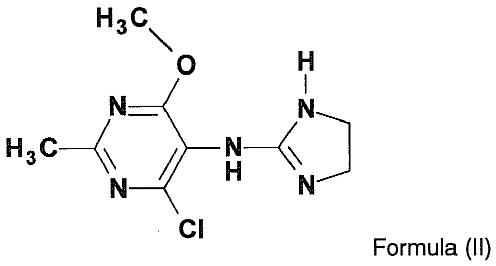 or a tautomer thereof, said process comprising:
- a step (i-c) of reacting a compound of Formula (IIa) 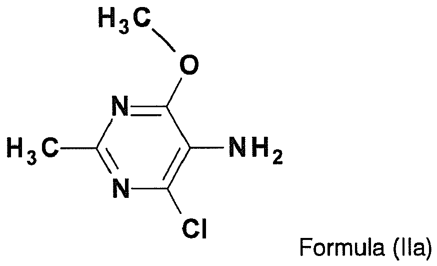 with a compound of formula (Ib) 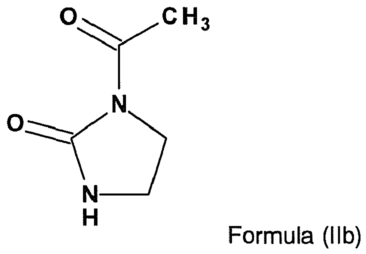 in dimethyl sulfoxide, in the presence of an effective amount of POCl₃ and an effective amount of anhydrous sodium carbonate;
- a step (ii-c) of directly reacting the product obtained in step (i-c) with a isopropanol-hydrochloride acid solution, thereby obtaining the chlorhydrate salt of the compound of Formula (II),
- a step (iii-c) of
o dissolving the product of step (ii-c) in water at a pH of about 9 thereby obtaining the product of step (ii-c) in a free base form, and subsequently extracting said free base with ethyl acetate and/or isopropyl acetate,
o subsequently dissolving said free base form in methanol at reflux and in the presence of activated carbon, and
o collecting crystals of compound of Formula (II) or a tautomer thereof.

12. A process for the manufacture of a crystalline form of a compound of Formula (II) or a tautomer thereof: 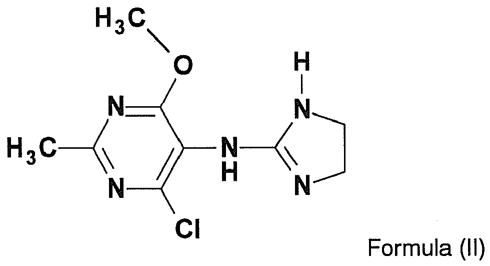 said process comprising the steps of
- dissolving a compound of Formula (II) in a C₁₋₄ aliphatic alcohol solvent or triturating the same in a ketone solvent, and
- collecting crystals of compound of Formula (II) or a tautomer thereof.

13. A compound having formula (Ic) 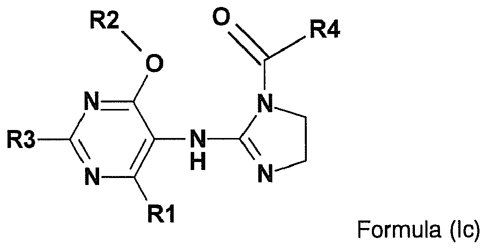 wherein R1, R2, R3 and R4 are as defined in claim 1.

14. The compound according to claim 13, having formula (IIc)

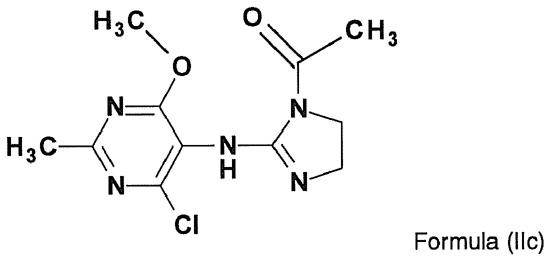

15. Use of a compound of formula (Ia) as intermediate in a process for the manufacture of a compound of Formula (I).
